# EUROPEAN PATENT SPECIFICATION

(11) **EP 1 648 403 B1**
(45) Date of publication and mention of the grant of the patent: **15.08.2012**
(21) Application number: 04733889.2
(22) Date of filing: 19.05.2004
(51) Int. Cl.: A61K 8/49, A61K 8/97, A61Q 19/06

(54) **SLIMMING COSMETIC COMPOSITION COMPRISING CAFESTOL OR KAHWEOL**
KOSMETISCHE SCHLANKHEITSMITTEL ENTHALTEND CAFESTOL ODER KAHWEOL
COMPOSITION COSMETIQUE AMINCISSANTE CONTENANT DU CAFESTOL OU DU KAHWEOL

(30) Priority: 20.05.2003 FR 0306063; 26.02.2004 JP 2004052321
(43) Date of publication of application: 26.04.2006
(73) Proprietor: Sederma, 78612 Le Perray-en-Yvelines Cedex (FR)
(72) Inventor: LINTNER, Karl, F-75015 Paris (FR); MAS CHAMBERLIN, Claire, 78460 CHEVREUSE (FR)
(86) International application number: PCT/IB2004/050755
(87) International publication number: WO 2004/103334

(56) References cited:
- FR-A- 2 479 688
- FR-A- 2 609 395
- FR-A- 2 733 149
- FR-A- 2 779 645
- US-A- 4 748 258
- US-A- 5 855 897
- DATABASE WPI Derwent Publications Ltd., London, GB; AN 1993-358162 XP002267772 & SU 1 770 352 A (AERO-R, STAL-R) 23 October 1992 (1992-10-23)
- RAUHEIM TRINE ET AL: "Effect of a coffee lipid (cafestol) on regulation of lipid metabolism in CaCo-2 cells" JOURNAL OF LIPID RESEARCH, vol. 36, no. 10, 1995, pages 2079-2089, XP002267771 ISSN: 0022-2275

## Description

### Technical Field

The present invention relates to the field of cosmetics and personal care products.

### Background Art

Cosmetic industry permanently researches for new ingredients which possess real activities and which are usable in a topical manner by everyone.

The unsightliness of 'spare tires' and localized fat deposits on thighs, long a preoccupation for women, has given rise to the development of numerous cosmetic slimming products. However, in order to accompany evolving attitudes in the overall management of localized fat overload problems, it is now necessary to address a wider public. In fact, while young and not so young women remain preoccupied by their figure, it is frequent for men to diet and practice body building targeted on the waist, hips and abdomen, zones that are particularly vulnerable to dietary relapses, a sedentary lifestyle and the cumulative effects of age.

Following the generation of slimming active substances based on direct activation of lipolysis *via* inhibition of phosphodiesterase (e.g. caffeine), more sophisticated products emerged. Those products address either stimulation of membrane receptors (G protein) or their inhibition (a -receptors and neuropeptide Y, NPY). Those approaches target activating glycerol release *via* adipocytic lipase (LHS). This approach induces depletion of cell lipid materials and hence a decrease in cell volume.

However, an innovative and complementary alternative route may exist: in addition to activating adipocytic fat store depletion, it is possible to deprive the cell of its supply source *via* the receptors enabling lipids to enter the cells. The adipocyte stores lipids supplied by the blood following ingestion of a meal using a special type of receptor: clathrin receptors.

Following a meal, cholesterol, triglycerides and other lipids are transported in the blood in the form of chylomicrons. Following partial hydrolysis, the chylomicrons yield various transport forms (see below), which constitute a system of addressing those forms to the various cell compartments (VALET and RICHARD Les lipides et la cellule adipeuse, NATHAN Editions, 1997).

For adipocytes, the address system mainly consists in LDL Low-density Lipoproteins) and VLDL Very Low-ensity Lipoproteins). We are now aware that adipocytes take in the nutrients required for maturation through the intermediary of LDL and VLDL. Triglyceride storage is largely related to the fatty acid released by VLDL (Yano et al., Atherosclerosis, 135 (1), p 57, 1997).

Following lipoprotein binding to clathrin receptors and endocytosis of the LDL receptor and VLDL receptor complexes, the triglyceride content and cholesterol transported are released in the adipocytes.

However, we discovered that the action of diterpenes like cafestol and kahweol as well as analogs and derivatives thereof on the regulation of the expression of the LDL and VLDL receptors allows to thwart lipidic storage of adipocytes while slowing down the development of neo-adipocytes. The document RANHEIM TRINE ET AL: "Effect of a coffee lipid (cafestol) on regulation of lipid metabolism in CaCo-2 cells" JOURNAL OF LIPID RESEARCH, vol. 36, no.10, 1995, pages 2079-2089, discloses that cafestol promotes uptake and degradation of LDL.
The document FR2609395 discloses the use of theophylline, theobromine and caffeine in cosmetic slimming preparations.

### Best Mode

The present invention relates to a topical cosmetic use according to claim 1.

The cosmetic compositions of the present invention contain at least one diterpene chosen among cafestol, kahweol, analogs and derivatives thereof. These are collectively referred to herein as 'diterpenes' unless the context or the explicit statements indicate otherwise. One or more additional ingredients, comprising one or more dermatologically acceptable carrier(s) are also preferably used in these compositions.

The term 'diterpenes' in accordance with the present invention is a compound of the following formula I:

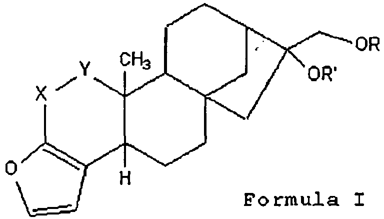

in which:

- R and R' groups, which can be identical or different, represent each one either an hydrogen atom, an alkyl group, an aryl group, an aralkyl group, an acyl group, a sulfonyl group, or a sugar group;

- The X-Y sequence corresponds either to CH₂-CH₂ or to CH=CH, or to CR¹R² - CR²R⁴, or also to CR⁵=CR⁶, in which the groups R¹, R², R³, R⁴, R⁵, R⁶, which can be identical or different, representing each one either an hydrogen atom, an alkyl group, an aryl group, an aralkyl group, an acyl group, a sulfonyl group, or a sugar group.

The topical compositions according to the present invention comprise at least one compound of the formula I.

The term 'alkyl group', in accordance with the present invention, means any alkyl group of 1 to 20 carbon atoms linear or branched, substituted or unsubstituted (particularly with an alcohol, a carboxylic acid, an amine), saturated or insaturated. A particularly preferred alkyl group is the methyl group.

The term 'aryl group', in accordance with the present invention, means one or several aromatic cycles possessing each one from 5 to 8 carbon atoms, which can be joined or amalgamate, substituted or not substituted. Particularly, aryl groups can be phenyl groups, or naphtyl and substituents can be halogen atoms, alkoxy groups like described above, alkyl groups like described above or the nitro group.

The term'aralkyl group', in accordance with the present invention, means any aryl group like abovedescribed, linked by an alkyl group like abovedescribed. A particularly preferred aralkyl group is the benzyl group.

The term'acyl group', in accordance with the present invention, means any group - C=OR⁷ in which R⁷ can be either an alkyl group, an aryl group, an aralkyl group, an amine group, like abovedescribed. A particularly preferred acyl group is the acetyl group (R⁷ = -CH₃).

The term 'amine group', in accordance with the present invention, means any group -NR⁸R⁹ in which R⁸ and R⁹, which can be which can be identical or different, represent each one either an hydrogen atom, an alkyl group, an aryl group, an aralkyl group, an acyl group, a sulfonyl group, or a sugar group like abovedescribed .

The term 'sulfonyl group', in accordance with the present invention, means any group -SO₂R¹⁰ in which R¹⁰ can be an alkyl group, an aryl group, an aralkyl group, an alkoxy group, an amine group, like abovedescribed. A particularly preferred sulfonyl group is the mesyl group (R¹⁰ = -CH₃), triflyl group (R¹⁰ = -CF₃), or tosyl group (R¹⁰ = - Ph-CH₃)_{.}

The term 'alkoxy group', in accordance with the present invention, means any group -OR¹¹ in which R¹¹ can be , an alkyl group, an aryl group, an aralkyl group, an acyl group, a sulfonyl group, or a sugar group like abovedescribed.

The term'sugar group', in accordance with the present invention, means any hexose group , ose group and oside group. Particularly preferred sugar groups are glucose groups, arabinose groups, fructose groups, galactose groups, mannose groups, maltose groups, lactose groups, saccharose groups, cellobiose groups.

Compounds in accordance with the present invention can possess several asymmetry centers and can exist then like optical isomers. The present invention relates to both isomers either separately, or as mixtures.

Particularly preferred compounds in accordance with the present invention are cafestol (formula I in which R = R' = H, X-Y = CH₂-CH₂), kahweol (formula I in which R = R' = H, X-Y = CH=CH), and cafestol acetate (formula Iin which R = - COCH₃, R' = H, X-Y = CH₂-CH₂).

Diterpenes like cafestol, kahweol, and analogs and derivatives thereof can be obtained either by chemical synthesis, or by plant extraction, from any plant that it is. A particularly preferred plant is coffee, and in particular green coffee bean, coffea Arabica (Coffee) Seed and coffea arabica bean. The extraction, from some plant specie or other, can be made according to a traditional extraction protocol.

Extraction solvents can be chosen among water, propylene glycol, butylene glycol, glycerin, PEG-6 Caprylic/capric glycerides, polyethylene glycol, methylic and/or ethylic diglycol ethers, cyclic polyols, ethoxylated or propoxylated diglycols, alcohols (methanol, ethanol, propanol, butanol), or any mixture of those solvents.

In addition, it is possible to make coffee extracts by other methods as, for example, steeping, simple decoction, lixiviation, extraction under reflux, supercritical extraction, extraction with ultrasounds or micro-waves or finally with countercurrent technology, without this list being restrictive.

The amount of diterpene used according to the invention depends on the effect sought and must be a safe and effective amount to provide slimming. For example, the diterpene of the present invention are preferably used in amounts from about 0.1 ppm (0.00001% w/w also referred to herein as 'weight percent', 'weight %' or simply by weight) to about 500,000 ppm (50% w/w), preferably from about 0.0001 % w/w (1 ppm) to 10% w/w, and most preferably from about 0.001 % w/w (10 ppm) to about 1% by weight of the composition.

In one preferred aspect of the present invention, there is provided the use of a cosmetic slimming composition which comprises at least a diterpene chosen among: cafestol, kahweol and analogs and derivatives thereof in a dermatologically acceptable carrier and optionally at least one lipolytic agent and/or an agent inhibiting lipogenesis, other than the aforementioned diterpene.

In order to complete the 'denutrition' of adipocytes, it is, possible to add a complementarity of effect by stimulating release of the triglycerides already resident in the cell.

The usual pathway of amplification of cAMP *via* inhibition of phosphodiesterase is the first intention pathway. Phosphodiesterase inhibitors include xanthic bases and natural plant extracts containing them. Xanthic bases are, for example, caffeine, analogs and derivatives thereof, theophylline analogs and derivatives thereof, theobromine, aminophylline, or xanthine. Those xanthic bases can be used alone or in association/mixtures. The natural plant extracts containing xanthic bases of the present invention, include tea extracts, coffee extracts, guarana extracts, mate extracts and cola extracts.

Other examples of lipolytic agents which may be used according to the present invention are some plant extracts which do not contain caffeine, and some marine extracts.

Besides phosphodiesterase inhibitors, the lipolytic agent may be chosen among: alpha-2 blockers compounds capable of blocking alpha-2 receptors at the adipocytes surface, beta-adrenergical agonists and antagonists, compounds stimulating beta receptors and/or G proteins, glucose transport blockers, neuropeptide Y (NPY) antagonists capable of blocking NPY receptors at the adipocytes surface, agents modifying fat acids transport, lipolytic peptides and lipolytic proteins.

A preferred embodiment according to the present invention is the association of at least a diterpene chosen among: cafestol, kahweol, and analogs and derivatives thereof, with at least a methyl xanthine chosen among theobromine and caffeine, analogs and derivatives thereof.

Methyl xanthines like abovedescribed can be obtained either by chemical synthesis, by plant extraction, from any plant it may be, and particularly from *Yerba mate.* In one preferred aspect of the present invention, at least a diterpene chosen among: cafestol, kahweol, and analogs and derivatives thereof, is combined with an extract of *Yerba mate,* a plant of the high Andean plateaus which is the source of a popular stimulant beverage rich in methyl xanthines. Among them, caffeine and theobromine are the most represented and their effect on stimulation of triglyceride release was investigated.

During the plant extraction process, any extraction solvent is available, including water, propylene glycol, butylene glycol, glycerin, PEG-6 Caprylic/capric glycerides, polyethylene glycol, methylic and/or ethylic diglycol ethers, cyclic polyols, ethoxylated or propoxylated diglycols, alcohols (methanol, ethanol, propanol, butanol), or any mixture of those solvents.

In addition, it is possible to make *Yerba Mate* extracts by other methods as, for example, steeping, simple decoction, lixiviation, extraction under reflux, supercritical extraction, extraction with ultrasounds or micro-waves or finally with countercurrent technology, without this list being restrictive.

Particularly preferred embodiments of the invention, include methyl xanthines from an extract of *Yerba Mate* titrated in caffeine and theobromine.

The amount of lipolytic or lipogenese inhibitor active ingredient, others than diterpene, present in the composition, can vary on a big range, and will be particularly from about 0.0001 % w/w to about 50% w/w, preferably from about 0.001 % w/w to 10% w/w, and most preferably from about 0.01 % w/w to about 1% by weight of the composition.

One preferred embodiment of the present invention is the association of an extract of green coffee beans/seeds rich in diterpenes (particularly cafestol and kahweol) and of an extract of Yerba Mate rich in methyl xanthines, and particularly caffeine, theobromine, aminophylline, théophylline, xanthine. This association provides a complementarity of effect which leads to a global reduction of fatty mass by a reduction of the adipocytes number and volume, by the blockage of LDL and VLDL internalisation by adipocytes and by stimulation of the release of the triglycerides already resident in the cell.

It has now been found that such an association of active ingredients in a composition, comprising at least a diterpene chosen among: cafestol, kahweol and analogs and derivatives thereof in a dermatologically acceptable carrier and at least one lipolytic agent and/or an agent inhibiting lipogenesis, other than the aforementioned diterpene can be synergistic. Particularly, the use in a composition of the invention of the combination of at least a diterpene chosen among: cafestol, kahweol, and analogs and derivatives thereof, with at least a methyl xanthine chosen among theobromine and caffeine, analogs and derivatives thereof, may show a synergy of slimming effect. And more particularly, the use in a composition of the invention of the combination of an extract of green coffee beans/seeds rich in diterpenes (particularly cafestol and kahweol) and of an extract of Yerba Mate rich in methyl xanthines, and particularly caffeine, theobromine, aminophylline, théophylline, xanthine shows a synergy in slimming effect.

According to a preferred embodiment of the invention, the slimming composition includes at least a complementary active ingredient chosen among:

- Actives acting on micro-circulation (vasculoprotectors or vasodilatators) like flavonoids, Gingko biloba extracts, ruscogenines, naturals or synthetic esculosides, escine extracted from horse chestnut, nicotinates, hesperidine methyl chalcone, the ruscus or butcher's-broom, lavender or rosemary essential oils, extracts of Ammi visnaga;

- Firming actives and/or anti-glycation actives (preventing sugar fixation on collagen fibers) as Centella asiatica extracts, Siegesbeckia extracts, silicium, amadorine, ergothioneine and derivatives, hydroxystilbenes and derivatives, for example resveratrol, plant extracts of the Ericaceae family, for exemple extracts of myrtille, vitamine C and derivatives, retinol and derivatives, protein derivatives such as Integrissyme® (offered by SEDERMA SAS France) or fermentation products such as Kombuchka®, also offered by SEDERMA.

- The compositions of the present invention may also contain a safe and effective amount of an anti-cellulite agent. Suitable agents may include, but are not limited to, xanthine compounds (*e.g*., caffeine, theophylline, theobromine, and aminophylline). Especially useful are combinations with the cellulite/slimming agents called Vexel (FR 2 654 619 of January 31, 1992), Coaxel (FR 2 694 195 of July 30, 1992), Cyclolipase (FR 2 733 149 of April 21, 1995), Pleurimincyl and Lipocare (WO 98/43607 of October 08, 1998) all offered by SEDERMA.

The amount of these complementary active ingredients may vary on a large scale. When present in the compositions of the present invention, the composition preferably contains from about 0.001% to about 50% w/w, more preferably from about 0.01 to 20% w/w, even more preferably from about 0.05% to about 10% w/w, still more preferably from 0,1% to about 5% by weight of the composition of the complementary active.

Particularly, the slimming composition of the invention may contain, besides the diterpene, an extract of *Yerba Mate,* and/or an extract of green coffee bean and/or a Coffea Arabica (Coffee) Seed extract.

Other active ingredients may be advantageously used within the context of the present invention. Specifically, the combination of the diterpenes that constitute the subject of the present invention with other cosmetic active substances *(vide infra)* is an advantageous implementation of the invention.

The term'dermatologically acceptable', as used herein, means that the compositions or components described are suitable for use in contact with human skin without risk of toxicity, incompatibility, instability, allergic response, and the like.

All terms such as 'slimming', 'skin firming', 'topical application', and the like are used in the sense in which they are generally and widely used in the art of developing, testing and marketing cosmetic and personal care products.

The present invention intention includes, but is not limited to, prevention and/or reduction of all outward visibly and tactilely perceptible manifestations as well as any other macro or micro symptoms noticed on the skin due to excess weight. As used herein, therapeutically regulating excess weight by a topical application of the topical composition of the present invention includes inducing a slimming treatment of weight overloads of the thighs and the hips, skin firming, and particularly preventing and/or treating cellulite or orange peel and/or refining contours of the face. It also includes reduction of steatomery of the thighs, reduction of the volume/fatty overload of the spare tire at the hips, reduction of 'love handles'. One have to notice that all those processes may happen both on men and women. The present invention has been found to act again excess weight both on men and women.

The term 'cosmetic composition' or 'topical composition' or more briefly just 'composition' in accordance with the present invention relates to a formulation that can be used for cosmetic purposes, purposes of hygiene or as a basis for delivery of one or more pharmaceutical ingredients. It is also possible that these formulations are used for two or more of these same purposes at one time. At a minimum, these compositions include a diterpene, analogs or derivatives thereof. These compositions may also include additional ingredients such as a dermatologically acceptable carrier.

'Cosmetics', as used herein, include without limitation, foundation, facial or body powder, sunscreens and blocks, mousse, sprays, whether in the form of creams, lotions, gels, ointments, emulsions, colloids, solutions, suspensions, compacts, solids, pencils, spray-on formulations, brush-on formulations and the like. 'Personal care products' include, without limitation, bath and shower gels, sunscreens and sunblocks, skin conditioners, cold creams, moisturizers, soaps, body scrubs, exfoliants, astringents, depilatories , antisweat and antiperspirant compositions, shaving, preshaving and after shaving products, moisturizers, deodorants, cold creams, cleansers, skin gels, rinses, whether in solid, powder, liquid, cream, gel, ointment, lotion, emulsions, colloids, solutions, suspensions, or other form. 'Pharmaceutical preparations' in accordance with the present invention include, without limitation, carriers for dermatological purposes, including topical and transdermal application of pharmaceutically active ingredients. These can be in the form of gels, patches, creams, nose sprays, ointments, lotions, emulsions, colloids, solutions, suspensions, powders and the like. Compositions in accordance with the invention include cosmetics, personal care products. The compositions are, for example, emollient lotions, milks or creams, milks or creams for skin care, makeup cleansing creams, lotions or milks, foundation bases, sunscreen lotions, sun milks or creams, artificial sun-tanning lotions, artificial sun-tanning milks or creams, shaving creams or foams, aftershave lotions.

The diterpenes compliant with the present invention, may be used in cosmetic compositions compliant with the invention either as the diterpenes themselves or in the form of a premix in a suitable excipient and they may be used in the form of a solution, dispersion, emulsion, paste or powder. They may individually or with other active substances, cited or not cited, be carried by cosmetic vectors such as macrocapsules, microcapsules or nanocapsules, macrospheres, microspheres or nanospheres, liposomes, oleosomes or chylomicrons, macroparticles, microparticles or nanoparticles, macrosponges, microsponges or nanosponges. They may also be adsorbed on powdered organic polymers, talcs, bentonites and other inorganic carriers.

The diterpenes may be used in any form or in a form that is bound, incorporated, absorbed in or adsorbed on macro-, micro- and nanoparticles, macro-, micro- and nanocapsules for the treatment of textiles, synthetic or natural fibers, wools and all materials liable to be used in the manufacture of clothing or underwear for the day or night, intended for contact with the skin, such as pantyhose, underwear, handkerchiefs and wipes, active materials of any sort, wipes, patches, compresses, cottons, cotton buds, dressings, makeup-removal sponges, masks and any other carrier liable to come into direct contact with the skin to enable continuous topical delivery in order to exert a cosmetic effect through the contact between the textile and skin.

Some of the compositions of the present invention may also provide additional benefits, including stability, absence of significant (consumer-unacceptable) skin irritation, anti-inflammatory activity and good aesthetics.

When the compositions compliant with the invention have the form of water in oil or oil in water emulsions, the oily phase mainly consists of a mixture of an extracted or synthetic fat with at least one oil and possibly another fat. The oily phase of the emulsions may constitute 5 to 60% of the total weight of the emulsion.

The aqueous phase of the said emulsions preferably constitutes 30 to 85% of the total weight of the emulsion. The proportion of emulsifier may be between 1 and 20% and preferably between 2 and 12% of the total weight of the emulsion. When these composition compliant with the invention are in the form of oily, oil in alcohol or dilute alcohol lotions, the compositions may constitute, for example, sunscreen lotions containing a filter absorbing UV radiation or softening lotions for the skin. Oily lotions may also constitute foaming oils containing an oil-soluble surfactant, bath oils, etc.

Among the main adjuvants that may be present in the compositions compliant with the invention are organic or hydroglycolic solvents, including MP-diol and polyglycerols, extracted or synthetic fats, ionic or non-ionic thickeners, softeners, opacifiers, stabilizers, silicones, α- or ß-hydroxyacids, vitamins, fragrances, preservatives, sequestering agents, colorants, gelling and viscosifying polymers, surfactants and emulsifiers, other water- or fat-soluble active substances, plant extracts, tissue extracts, marine extracts, sun filters and antioxidants, moisturizing agents, softening agents, products for the treatment of skin diseases, germicides, propellants.

The most preferred mono- or poly-alcohols are selected from ethanol, isopropanol, propylene glycol, glycerol and sorbitol. As the fat, among the mineral oils, may be cited liquid paraffin, among the animal oils, whale, shark, seal, menhaden, haddock liver, cod, tuna, tortoise, calf's foot, horse's foot, sheep's foot, mink, otter and marmot oil, etc. Among the plant oils may be cited almond, wheat germ, jojoba, sesame, sunflower, palm, Brazil nut, shea, shorea, macadamia, blackcurrant seed and similar oils.

Among the fatty acid esters may be used C₁₂ to C₂₂ acid esters, saturated or unsaturated, and lower alcohols such as isopropanol and glycerol, or C₈ to C₂₂ acids, linear or branched, saturated or unsaturated, or 1,2-alkanediols of C₁₀ to C₂₂.

Other fats include liquid paraffin, paraffin, lanolin, hydrogenated lanoline, tallow, acetylated lanoline and silicone oils.

Among the waxes may be cited Sipol wax, lanoline wax, beeswax, Candelila wax, monocrystalline wax, Carnauba wax, spermaceti, cocoa butter, shea butter, silicone waxes, hydrogenated oils solid at 25°C, sucroglycerides, oleates, myristates, linoleates and calcium, magnesium and aluminum stearates.

Among the fatty alcohols may be cited lauric, cetyl, myristic, stearic, palmitic and oleic alcohol and Guerbet alcohols such as 2-decyltetradecanol or 2-hexyldecanol. As emulsifiers, among the polyoxyethylenated fatty alcohols may be cited lauric, cetyl, stearyl and oleic, containing 2 to 20 moles of ethylene oxide, alcohols. Among the alkyl ethers may be cited glycerol and C₁₂-C₁₈ alcohols with 2 to 10 moles of glycerol. Thickeners may also be of value, such as cellulose derivatives, polyacrylic acid derivatives, guar gum, ceratonia and xanthan gum.

When the compositions compliant with the invention are dispersions, they may consist of dispersions of lecithin in water in the presence of a surfactant or aqueous dispersions of lipid spherules consisting of organized molecular layers enclosing an encapsulated aqueous phase. In that context, as lipid compounds, may be cited long-chain alcohols and diols, sterols such as cholesterol, phospholipids, cholesteryl sulfate and phosphate, long-chain amines and quaternary ammonium derivatives, dihydroxyalkylamines, polyoxyethylenated fatty amines, long-chain aminoalcohol esters, their salts and quaternary ammonium derivatives, phosphoric esters of fatty alcohols such as acid dicetylphosphate or its sodium salt, alkylsulfates such as sodium cetylsulfate, fatty acids in the form of salts or lipids of the type described in French patent No. 2 315 991, No. 1477 048 and No. 2 091516 or in international patent applications WO 83/01 571 and WO 92/08685.

As examples of other lipids that may be used may be cited lipids carrying a long lipophilic chain of 12 to 30 carbon atoms, saturated or unsaturated, branched or linear, for example, an oleic, lanolic, tetradecylic, hexadecylic, isostearylic, lauric or alkylphenylic chain. The hydrophilic group of those lipids may be an ionic or non-ionic group. As examples of non-ionic groups may be cited those derived from polyethylene glycol. As lipids forming the lamellar phase, the following may advantageously be used: polyglycerol ethers such as those described in French patents No. 1 477 048 , No. 2 091516, No. 2 465 780 and No. 2 482 128.

As an ionic group, a group derived from an amphoteric, anionic or cationic compound may advantageously be used.

Other lipids described in the international patent application, WO 83/01 571, as suitable for use in the formation of vesicles are glycolipids such as lactosylceramide, galactocerebroside, gangliosides and trihexosylceramide together with phospholipids such as phosphatidyl glycerol and phosphatidyl inositol.

Additional Ingredients

In addition to the diterpene, analogs and/or derivatives thereof described herein, the composition of the invention may include various other and additional ingredients, which may be active, functional, conventionally used in cosmetic, personal care or topical/transdermal pharmaceutical products or otherwise, which provide some benefit to the object of the composition. Such additional ingredients may include one or more substances such as, without limitations, cleaning agents, skin conditioning agents, perfumes, sunscreen and/or sunblock compounds for skin, detergents, pharmaceuticals, humectants, emollients, antiseptic agents, deodorant actives.

The compositions of the present invention generally contain at least one additional ingredient. The compositions of the present invention may contain a plurality of additional ingredients as well. Usually these compositions include at least one dermatologically acceptable carrier.

In a preferred embodiment, where the composition is to be in contact with human keratinous tissue, the additional ingredients should be suitable for application to keratinous tissue, that is, when incorporated into the composition they are suitable for use in contact with human keratinous tissue (skin) without undue toxicity, incompatibility, instability, allergic response, and the like within the scope of sound medical judgment. The CTFA Cosmetic Ingredient Handbook, Ninth Edition (2002) describes a wide variety of nonlimiting cosmetic and pharmaceutical ingredients commonly used in the skin care industry, which are suitable for use as additional ingredients in the compositions of the present invention. Non-limiting examples of these additional ingredient classes include: abrasives, absorbents, aesthetic components such as fragrances, pigments, colorings/colorants for hair and skin, makeup agents, essential oils, skin sensates, astringents, etc. (*e.g*., clove oil, menthol, camphor, eucalyptus oil, eugenol, menthyl lactate, witch hazel distillate), anti-acne agents, anti-caking agents, antifoaming agents, antimicrobial agents (*e.g*., iodopropyl butylcarbamate), binders, biological additives, buffering agents, bulking agents, chelating agents, chemical additives, cosmetic astringents, cosmetic biocides, denaturants, drug astringents, external analgesics, film formers or materials, *e.g*., polymers, for aiding the film-forming properties and substantivity of the composition ( *e.g*., copolymer of eicosene and vinyl pyrrolidone), opacifying agents, pH adjusters, propellants, reducing agents, sequestrants, skin bleaching and lightening agents (*e.g*., hydroquinone, kojic acid, ascorbic acid, magnesium ascorbyl phosphate, ascorbyl glucosamine), skin-conditioning agents (*e.g*., humectants, including miscellaneous and occlusive), skin soothing and/or healing agents (*e.g*., panthenol and derivatives (*e.g.,* ethyl panthenol), aloe vera, pantothenic acid and its derivatives, allantoin, bisabolol, and dipotassium glycyrrhizinate), skin treating agents, thickeners, peptides and peptide derivatives thereof, vitamins and derivatives thereof.

The active substances which may be combined with the diterpenes, may be substances of pharmaceutical or dietary value or be endowed with their own cosmetic activity. When those active substances are water-soluble, they may be uniformly dissolved or they may be dissolved in an encapsulated aqueous phase inside vesicles. The water-soluble substances with cosmetic and/or pharmaceutical activity may be products intended for care or treatment of the skin ; such as, for example, wetting agents like glycerol, sorbitol, pentaerythritol, pyrrolidone acid and its salts; artificial sun-tanning agents such as dihydroxyacetone, erythrulose, glyceraldehyde, and γ-dialdehydes such as tartaraldehyde, those compounds possibly being combined with colorants; water-soluble sunscreens; antiperspirants, deodorants, astringents, and refreshing, tonic, cicatrizing, keratolytic and depilatory products, perfumed water; botanical extracts, plant tissue extracts such as polysaccharides; water-soluble colorants; antiseborrheic agents, oxidants such as bleaching agents such as hydrogen peroxide; reducing agents such as thioglycolic acid and its salts.

The following may also be cited: vitamins B through B₁₂, C, D, H, K and their derivatives, peptide or steroid hormones, enzymes (*e.g*., superoxide dismutase), coenzymes, enzyme inhibitors, enzyme activators, vaccines, steroidal or non-steroidal anti-inflammatories such as hydrocortisone, antibiotics, antimicrobial and bactericidal substances, cytotoxic or antineoplastic agents

When the active substances are fat soluble, they may be incorporated in the lipid phase of the emulsions or in the membranes of vesicles such as liposomes, micelles and chylomicrons. The active substances may be selected in the group formed by fat-soluble active substances selected from the group formed by the fat-soluble sunscreens, substances intended to improve the state of dry or aged skin, tocopherols, vitamins E, F or A and their esters, retinoic acid, antioxidants, essential fatty acids, glycyrrhetinic acid, keratolytics and carotenoids, ceramides and pseudo-ceramides, and all lipid complexes of a form similar to that of the natural ceramides of the skin.

The present invention also relates to the use, by topical application, of a cosmetic composition according to the invention, in order to prevent and/or reduce all outward visibly and tactilely perceptible manifestations as well as any other macro or micro symptoms noticed on the skin due to excess weight, to induce a slimming treatment of weight overloads of the thighs and the hips, skin firming, and particularly to prevent and/or treat cellulite or orange peel and/or refine contours of the face, to reduce the steatomery of the thighs, the volume/fatty overload of the spare tire at the hips, to reduce 'love handles', both on men and women.

The present invention also relates to the use of a composition according to the invention, like or for the manufacturing of a cosmetic composition for a slimming treatment of weight overloads of the thighs and the hips, skin firming, and particularly prevent and/or treat cellulite or orange peel and/or to refine contours of the face.

The present invention concerns a cosmetic treatment in order to prevent and/or treat cellulite and/or orange peel and/or refine contours of the face, comprising a topical application of a cosmetic composition according to the invention.

The composition of the invention can be locally applied on the parts of the face or of the body which need such a treatment, particularly on hips, buttocks, thighs, the belly, and the oval of the face. One of the advantages of the present invention is in the possibility of being available to proceed, each time it is necessary or desirable, to 'soft' treatments very localized and selective thanks to the application way by topical route.

The present invention concerns also the use of a composition of the invention to make cosmetics, personal care products, intended for a slimming treatment of excess weight of the thighs and the hips, skin firming, and particularly prevent and/or treat cellulite or orange peel and/or to refine contours of the face.

### Mode for Invention

The following examples further describe and demonstrate embodiments within the scope of the present invention. The examples are given solely for the purpose of illustration and are not to be construed as limitations of the present invention,

As an illustration of the invention, several cosmetic formulae will be cited. The formulae are representative of, but do not restrict, the invention:

Example 1 : Slimming Preparation (SP1)

| **Ingredients** | **g/100g** |
|---|---|
| *Yerba Mate* extract | 30.0 |
| Butylene glycol | 15.0 |
| Green coffee beans extract | 10.0 |
| Crovol A70 | 5.0 |
| Glycerin | 5.0 |
| Cetyl hydroxyethylcellulose | 1.5 |
| Water, preservatives | qsp 100 g |

This preparation SP1 guarantees the following active quantities:

| Active ingredient | % w/w of SP1 |
|---|---|
| Cafestol | 0.05-0.07% |
| Caffeine | 0.9-1% |
| Theobromine | 0.09-0.11% |

This preparation, SP1, is one preferred form of the invention. The amount of this preparation in a cosmetic composition may vary in a large scale and will be preferably between around 0.001% and around 20% w/w, more preferably between around 0.01% and around 10% w/w, by the total weight of the cosmetic composition. Preparation SP1 may be the slimming active ingredient of the following examples.

Example 2 : Slimming Gel

| **Ingredients** | **g/100 g** |
|---|---|
| Carbopol 1342 | 0.3 |
| Propylene glycol | 2.0 |
| Glycerin | 1.0 |
| White paraffine | 1.5 |
| Cylomethicone | 6.0 |
| Cetylic alcohol | 0.5 |
| Lubrajel MS | 10.0 |
| Triethanolamine | 0.3 |
| SP1 | 3.0 |
| Water, preservatives, fragrance | qsp 100 g |

Example 3 : Massage slimming cream

| **Ingredients** | **g/100 g** |
|---|---|
| Ultrez 10 | 0.2 |
| Butylene glycol | 5.0 |
| Stearic acid | 1.5 |
| Crodamol GTCC | 2.0 |
| Petrolatum oil | 2.0 |
| Crodacol C90 | 0.5 |
| Crodafos CES | 1.5 |
| SP1 | 3.0 |
| Water, preservatives, fragrance | qsp 100 g |

Example *4 : in vitro* studies: Effect of cafestol acetate on the mRNA of LDLr and VLDLr receptors

To evidence regulation of transcription (control of the number of copies of the messenger RNA for the gene coding for the protein studied), it was necessary to isolate the messenger RNA (**mRNA**) synthesized from the cell culture. The mRNA specific for the protein of interest must be recognized in the total mRNA pool present in the cell.

The used method is RT-PCR, of amplification and quantization, conducted in real time on the latest-generation systems and which enables in-line quantitative measurement of the target mRNA.

*Protocol*

Neo-adipocytes 3T3-L1 in the adipocytic differentiation phase (after addition of an appropriate triggering cocktail) were incubated with cafestol acetate for 24 hours.

At the end of the test, the adipocytes were lysed and the extracted mRNA transcribed to cDNA (by reverse transcriptase). The cDNA were then amplified (n copies using Taq polymerase) to obtain a detectable signal.

The amplicons (cDNA amplification products) were quantified directly, as they emerged in the RT-PCR system, then subjected to electrophoresis in order to visually illustrate the results.

*Results*

Table 1: Quantification of mRNA expression in adipocytes incubated with cafestol acetate for 24 hours. Values standardized on the internal standard, b -actin, were then expressed as percentages relative to the control. Mean results for n = 3 tests

| | | |
|---|---|---|
| | mRNA expression: Values standardized on the internal standard | |

| | VLDL receptor | LDL receptor |
|---|---|---|
| Control | 100 | 100 |
| Cafestol Acetate 15ppm | 90 | 66 |

Cafestol acetate showed a very marked effect, decreasing mRNA expression of the LDL receptor. The reduction was -33%, equivalent to that of the positive control, and 10% of reduction of the VLDLr in 24 hours.

Example 5 : *In vivo* studies

BODY CREAM formula used for the clinical study on the female panel:

| **Ingredients** | **g/100 g** |
|---|---|
| Ultrez 10 | 0.4 |
| Glycerin | 10.0 |
| Crillet 1 | 2.0 |
| Crodamol OP | 4.0 |
| Dimethicone | 3.0 |
| Potassium sorbate | 0.1 |
| NaOH 38% | 0.6 |
| Ethanol | 3.0 |
| SP1 | 3.0 |
| Water, preservatives, fragrance | qsp 100 g |

MASSAGE CREAM formula used for the clinical study on the male panel:

| **Ingredients** | **g/100 g** |
|---|---|
| Ultrez 10 | 0.2 |
| Buthylene glycol | 5.0 |
| Crodamol GTCC | 2.0 |
| Crodamol C90 | 0.5 |
| Dimethicone | 1.5 |
| DC345 | 2.0 |
| Potassium sorbate | 0.1 |
| Stearic acid | 1.5 |
| NaOH 38% | 0.5 |
| Ethanol | 3.0 |
| SP1 | 3.0 |
| Water, preservatives, fragrance | qsp 100 g |

The *in vivo* efficacy study on SP1 was conducted using a female panel and a male panel. The method known as interference-fringe topometry was used. The method consists in analysis of the optical deformation of fringe projection under laser illumination (Fast Optical In vivo Topometry of human Skin, FOITS).

*Principle*

The subjects are placed at a precise distance and in a position that was perfectly reproducible from one measurement to the next. The zones under analysis are successively scanned by fringes of different widths. The measurements are conducted on a site precisely located in terms of natural features such as beauty spots and visible venules.

The deformation related to the state of the surface scanned is automatically recorded for increasingly narrow projected fringes. The automatic signal acquisition using a CCD camera enables reconstitution of the volume explored without direct contact with the subject. The volume explored is expressed in mL and the changes in volume between the start and end of the study enable quantitation of the local loss.

*Protocol*

The volunteers, 15 male subjects and 12 female subjects, applied the cream twice daily for 56 days. Each subject acted as his/her own control and the treated side was compared with the untreated contralateral side. So doing eliminated any contextual variation in volume independent of the effect studied during the study.

Study on the female panel

*Inclusion criteria:*

Women aged 18 to 45 years, Caucasian, with steatomery of the thighs, BMI between 20 and 26 kg/m², weight stable for at least 3 months, no slimming diet in the 3 months preceding the study.

*Results:*

The 3D acquisitions enabled reconstruction of thigh volume between 2 inferior and superior levels. Twelve volunteers of mean age 28.7 ± 8 years took part in the study. Thigh volume was compared at T0 and T56. The thigh perimeter parameter is given for the superior transverse section, the upper volume cutoff. At T0, there was no significant difference between the volumes of the right and left thighs. The mean changes in volume after 56 days were determined and the significance tested using Student's t test for paired series (treatment vs. control) and an unpaired t test (difference: T0 vs. T56 for each thigh).

Table 2: Mean change in thigh volume, T0 vs. T56 days, after twice daily application of 3% SP1 by a panel of 12 women

| FEMALE PANEL | Treated | Treated | Untreated | Untreated |
|---|---|---|---|---|
| | T0 | T56 | T0 | T56 |
| Volume (mL) | 2662 | 2391 | 2512 | 2564 |
| % change | - | -10.07% | - | +0.2% |
| Maximum % change | | -27% | | - |
| Significance / T0 | | p<0.01 | | NS |
| Significance vs. untreated | | p<0.05 | | |

Table 3: Mean change in thigh perimeter, T0 vs. T56 days, after twice daily application of 3% SP1 by a panel of 12 women.

| FEMALE PANEL | Treated | Treated | Untreated | Untreated |
|---|---|---|---|---|
| | T0 | T56 | T0 | T56 |
| Perimeter (cm) | 57.27 | 56.50 | 57.29 | 57.38 |
| Change | | -0.8 cm | | +0,1 cm |
| Maximum change | | 1.9 cm | | - |
| Significance / T0 | | p<0.01 | | NS |
| Significance vs. untreated | | p<0.05 | | |

After 56 days of application, thigh volume and perimeter showed very significant changes with a 10% decrease in volume (p<0.01) and a reduction in thigh perimeter of nearly 1 cm (p<0.01). Over the same period, there was no significant difference for the untreated thighs and even a non-significant very slight increase. Daily use of 3% SP1 thus demonstrated its ability to reduce fat overload of the thighs in women.

Study on the male panel

*Inclusion criteria*

Subjects aged ³ 30 years, Caucasian, BMI between 20 and 26 kg/m², presence of fat overloads in the form of visible spare tire at the hips, weight stable for at least 3 months, no slimming diet in the 3 months preceding the study.

*Results*

The 3-D acquisitions enabled reconstruction of the lateral surface and volume of the spare tire on the hips. In this case, the accessible parameter was a fictitious volume (since, in contrast to what was done with the thigh, only the lateral contour can be explored) taking into account the mean distance separating the surface of the spare tire and a fictional section plane (describing a section of the thorax) that was precisely positioned. Accordingly, for each subject, the mean variation in the amplitude of the spare tire was determined in mL and could not be converted to a percentage.

Fifteen volunteers of mean age 42.3 ± 11 years took part in the study. At T0, there was no significant difference between the volume of the spare tire on the right and left. The mean changes in volume of the projecting part of the hip after 56 days were determined and the significance calculated using Student's t test for paired series (treatment vs. control) and unpaired series (difference, T0 vs. T56, for each hip).

Table 4: Mean changes in spare tire amplitude, T0 vs. T56 days, after twice daily application of 3% SP1 by 15 male volunteers.

| MALE PANEL | Treated side | Untreated side |
|---|---|---|
| Volume change (mL) | -17.7 | -4.2 |
| Significance /T0 | p<0.059 | NS |

After 56 days of application, the volume of the spare tire at the hip decreased by 17.7 mL. Over the same period, there was no significant difference for the untreated hip. Used daily, 3% SP1 demonstrated its ability to reduce fatty overload of the hips (spare tire) in men.

Example 6 : Slimming anti-stretchmark gel

| **Ingredients** | **INCI** | | **% by wt.** |
|---|---|---|---|
| Part A | | | |
| Water Deionised | Water (Aqua) | | qsp 100% |

| Part B | | | |
|---|---|---|---|
| Butylene Glycol | | | 5.00 |
| Phenova | Phenoxyethanol (and) Mixed Parabens | Crodarom | 0.80 |

| *Part C* | | | |
|---|---|---|---|
| Crill 3 | Sorbitan Stearate | | 1.20 |
| Crillet 3 | Polysorbate 60 | Croda | 3.00 |
| DC 200 | Dimethicone | Dow Corning | 2.00 |
| Crodamol IPM | Isopropyl Myristate | Croda | 5.00 |
| Crodamol W | Stearyl Heptanoate | | 0.30 |
| Crodamol GTCC | Caprylic/Capric Triglyceride | | 5.00 |
| Crodacol CS90 | Cetearyl Alcohol | | 2.00 |
| Ceramide 2 | N-stearoylsphingani ne | SEDERMA | 0.10 |

| *Part D* | | | |
|---|---|---|---|
| Carbopol 980 at 2% | Carbomer | BF Goodrich | 10.00 |

| *Part E* | | | |
|---|---|---|---|
| Potassium Sorbate | | | 0.10 |

| *Part F* | | | |
|---|---|---|---|
| Water Deionised | | | 2.00 |
| Sodium Hydroxide | | | 0.20 |

| *Part G* | | | |
|---|---|---|---|
| Water | | | 10.0 |
| DERMAXYL® | | SEDERMA | 3.00 |
| Rutin | | | 0.1 |
| Bowman Birk | | | 0.0001 |
| Inhibitor | | | |
| UNISLIM® | | SEDERMA | 3.00 |

UNISLIM® : Ilex Paraguariensis (Leaf) Extract (and) Water (and) Butylene Glycol (and) Coffea Arabica (Coffee) Seed Extract (and) PEG-60 Almond Glycerides (and) Glycerin (and) Cetyl Hydroxyethylcellulose.

DERMAXYL® : C12-15 Alkyl Benzoate(and) Tribehenin (and) Ceramide 2 (and) PEG-10 Rapeseed Sterol (and) Palmitoyl Oligopeptide

This gel can be prepared in the following way: Homogenize Part B and pour it into Part A. Heat Part (A+B) to 75°C. Heat Part C and Part D to 75°C. Pour Part C into Part (A+B) with helix stirring; then, pour Part D into Part (A+B+C). Add Part F and Part E. Pour Part G at about 35°C.

Example 7: Slimming anti Stretch-Mark Cream

| **Ingredients** | **INCI** | | **% by wt.** |
|---|---|---|---|
| Part A | | | |
| Water Deionised | Water (Aqua) | | qsp 100% |
| Ultrez 10 | Carbomer | Noveon | 0.40 |

| Part B | | | |
|---|---|---|---|
| Glycerin | | Croda | 5.00 |
| Phenova | Phenoxyethanol (and) Mixed Parabens | Crodarom | 0.80 |

| *Part C* | | | |
|---|---|---|---|
| Crodamol OP | Ethylhexyl Palmitate | Croda | 4.00 |
| Crodacol CS90 | Cetearyl Alcohol | Croda | 0.50 |
| Crodamol ML | Myristyl Lactate | Croda | 0.30 |
| Crillet 1 | Polysorbate 20 | Croda | 1.00 |

| *Part D* | | | |
|---|---|---|---|
| Pemulen TR2 | Acrylates/C 10-30 Alkyl Acrylate (and) Crosspolymer | Noveon | 0.20 |
| DC 345 | Cyclomethicone | Dow Corning | 2.00 |

| *Part E* | | | |
|---|---|---|---|
| Potassium Sorbate | | | 0.10 |

| *Part F* | | | |
|---|---|---|---|
| Water Deionised | | | 6.00 |
| Sodium Hydroxide | | | 0.60 |

| *Part G* | | | |
|---|---|---|---|
| MATRIXYL® 3000 | | SEDERMA | 3.00 |
| Darutoside | *Siegesbeckia Orientalis* Extract | SEDERMA | 2.00 |
| UNISLIM® | | SEDERMA | 3.00 |

Darutoside is a molecule sold by SEDERMA for the treatment of stretch marks.

MATRIXYL® 3000 is sold by SEDERMA for the treatment of wrinkles [Glycerin (and) Water *(Aqua)* (and) Butylene Glycol (and) Carbomer (and) Polysorbate 20 (and) Palmitoyl Oligopeptide (and) Palmitoyl Tetrapeptide-3].

UNISLIM® : Ilex Paraguariensis (Leaf) Extract (and) Water (and) Butylene Glycol (and) Coffea Arabica (Coffee) Seed Extract (and) PEG-60 Almond Glycerides (and) Glycerin (and) Cetyl Hydroxyethylcellulose.

This emulsion is prepared in the following way : Phase A : disperse Ultrez 10 in water and let it swell for 20 minutes, then add phase B; heat to 75 C°. Heat Phase C separately to 75°C. Mix the two phases under stirring, homogenise, add Phase D, neutralise with Phase E, cool until reaching 30°C, then add Phase F and Phase G, adjust pH to -6 with NaOH.

### Industrial Applicability

The present invention relates to the cosmetic and personal care industries.

## Claims

1. Topical cosmetic use of at least one diterpene chosen among cafestol, kahweol, analogs and derivatives thereof, for a slimming treatment of excess weight of the thighs and the hips, skin firming, preventing and/or treating cellulite or orange peel and/or refining contours of the face.

2. The use according to claim 1, wherein said diterpene corresponds to formula I : 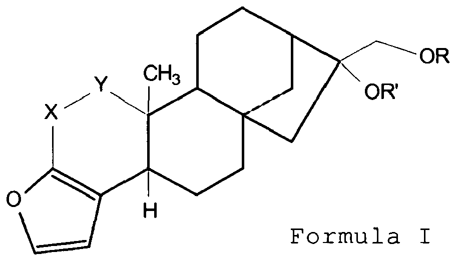 in which:
- R and R' groups, which can be identical or different, represent each one either an hydrogen atom, an alkyl group, an aryl group, an aralkyl group, an acyl group, a sulfonyl group, or a sugar group;
- The X-Y sequence corresponds either to CH2-CH2 or to CH=CH or to CR1R2-CR2R4, or also to CR5=CR6, in which the groups R1, R2, R3, R4, R5, R6, which can be identical or different, representing each one either an hydrogen atom, an alkyl group, an aryl group, an aralkyl group, an acyl group, a sulfonyl group, or a sugar group.

3. The use according to claim 1 or 2, wherein said diterpene is obtained from a plant extract.

4. The use according to claim 3, wherein said plant extract is a green coffee seed extract.

5. The use according to anyone of claims 1 to 4, further comprising at least one lipolytic agent and/or an agent inhibiting lipogenesis, other than the diterpene.

6. The use according to claim 5, wherein said lipolytic agent is chosen among: phosphodiesterase inhibitors, α-2 blockers compounds, β-adrenergical agonists and antagonists, compounds stimulating β receptors and/or G proteins, glucose transport blockers, neuropeptide Y (NPY) antagonists capable of blocking NPY receptors at the adipocytes surface, agents modifying fat acid transport, lipolytic peptides and lipolytic proteins.

7. The use according to claim 6, wherein said phosphodiesterase inhibitor is chosen among: xanthic bases (caffeine, theobromine, aminophylline, theophylline, xanthine, analogs and derivatives thereof) and natural plant extracts containing them, and in particular methyl xanthines.

8. The use according to claim 7, wherein said phosphodiesterase inhibitor is caffeine and/or theobromine.

9. The use according to anyone of claims 1 to 8, wherein said diterpene is present in an amount between about 0.00001% and about 50% w/w, preferably from about 0.0001% to about 10% w/w, and most preferably from 0,001% to about 1% by weight of the composition.

10. The use according to anyone of claims 5 to 9, wherein said lipolytic agent and/or agent inhibiting lipogenesis is present in an amount between about 0.0001% and about 50% w/w, preferably from about 0.001% to about 10% w/w, and most preferably from 0,01% to about 1% by weight of the composition.

11. The use according to claim 7 or 8, wherein said natural plant extract comprising xanthic bases is a Yerba Mate extract.

12. The use according to anyone of claims 1 to 11, further comprising at least complementary active ingredient chosen among: actives acting on micro-circulation, firming actives and/or anti-glycation actives and their mixtures.

13. The use according to anyone of claims 1 to 12, wherein said diterpene is used in the form of a solution, dispersion, emulsion, paste or powder, individually, or in a premix, or is carried individually or in a premix by vectors such as macrocapsules, microcapsules or nanocapsules, macrospheres, microspheres or nanospheres, liposomes, oleosomes or chylomicrons, macroparticles, microparticles or nanoparticles, macrosponges, microsponges or nanosponges, or are adsorbed on powdered organic polymers, talcs, bentonites or other inorganic carriers.

14. The use according to anyone of claims 1 to 13, wherein said diterpene is used individually, or in a premix in an acceptable carrier in any cosmetic form, namely emollient lotions, milks or creams, milks or creams for skin care, makeup cleansing lotions or milks, foundation bases, sunscreen lotions, sun milks or creams, artificial suntanning lotions, artificial suntanning milks or creams, shaving creams or foams or aftershave lotions.

15. The use according to anyone of claims 1 to 14, comprising at least one additional ingredient selected from the following: organic or hydroglycolic solvents, extracted or synthetic fats, ionic or non-ionic thickeners, softeners, opacifiers, stabilizers, silicones, α- or ß-hydroxyacids, preservatives, sequestering agents, gelling and viscosifying polymers, surfactants and emulsifiers, plant extracts, tissue extracts, marine extracts, sun filters, moisturizing agents, softening agents, products for the treatment of skin diseases, germicides, propellants, cleaning agents, skin conditioning agents, perfumes, sunscreen and/or sunblock compounds for skin, detergents, emollients, antiseptic agents, abrasives, absorbents, aesthetic components such as fragrances, pigments, colorings/colorants for skin, make-up agents, essential oils, skin sensates, astringents, anti-acne agents, anti-caking agents, antifoaming agents, antimicrobial agents, inders, biological additives, buffering agents, bulking agents, chelating agents, chemical additives, cosmetic astringents, cosmetic biocides, denaturants, drug astringents, external analgesics, film formers or materials, pH adjusters, reducing agents, skin bleaching and lightening agents, humectants, skin soothing and/or healing agents, skin treating agents, peptides and peptide derivatives thereof, vitamins and derivatives thereof, wetting agents, artificial sun-tanning agents; water-soluble sunscreens; antiperspirants, deodorants, and refreshing, tonic, cicatrizing, keratolytic and depilatory products, perfumed water, botanical extracts, plant tissue extracts, water-soluble colorants, antiseborrheic agents, oxidants, vitamins B1 through B12, C, D, H, K and their derivatives, peptide or steroid hormones, enzymes, coenzymes, enzyme inhibitors, enzyme activators, bactericidal substances, fat-soluble active substances, substances intended to improve the state of dry or aged skin, tocopherols, vitamins E, F or A and their esters, retinoic acid, antioxidants, essential fatty acids, glycyrrhetinic acid, carotenoids, ceramides and pseudo-ceramides, and all lipid complexes of a form similar to that of the natural ceramides of the skin.

16. The use according to anyone of claims 1 to 15, for the impregnation of any sort of textile, synthetic or natural fiber, wool or any material liable to be used for the manufacture of clothing or underclothing, active materials of any sort, wipes, patches, compresses, cottons, cotton buds, dressings, makeup-removal sponges, masks and any other carrier liable to come into direct contact with the skin to enable continuous topical delivery.

## Patentansprüche

1. Topische kosmetische Verwendung wenigstens eines Diterpens, ausgewählt aus Cafestol, Kahweol, Analogons und Derivaten solcher, zur Schlankheitsbehandlung von Übergewicht der Schenkel und der Hüften, Festigung der Haut, Vorbeugung und/oder Behandlung von Cellulitis oder Orangenhaut und/oder Verfeinerung von Gesichtskonturen.

2. Verwendung nach Anspruch 1, wobei das Diterpen auf Formel I bezogen ist: 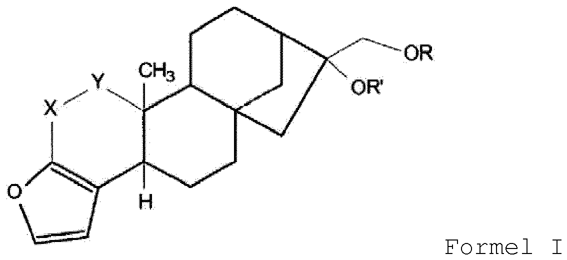 wobei:
- die Gruppen R und R', welche identisch oder verschieden sein können, jeweils entweder ein Wasserstoffatom, eine Alkylgruppe, eine Arylgruppe, eine Aralkylgruppe, eine Acylgruppe, eine Sulfonylgruppe oder eine Zuckergruppe darstellen;
- die Sequenz X-Y entweder CH2-CH2 oder CH=CH oder CR1R2-CR2R4 oder auch CR5=CR6 entspricht, wobei die Gruppen R1, R2, R3, R4, R5, R6, welche identisch oder verschieden sein können, jeweils entweder ein Wasserstoffatom, eine Alkylgruppe, eine Arylgruppe, eine Aralkylgruppe, eine Acylgruppe, eine Sulfonylgruppe oder eine Zuckergruppe darstellen.

3. Verwendung nach Anspruch 1 oder 2, wobei das Diterpen aus einem Pflanzenextrakt gewonnen wurde.

4. Verwendung nach Anspruch 3, wobei der Pflanzenextrakt ein Rohkaffeeextrakt ist.

5. Verwendung nach einem der Ansprüche 1 bis 4, weiter umfassend wenigstens ein lipolytisches Mittel und/oder ein Lipogenese inhibierendes Mittel anders als das Diterpen.

6. Verwendung nach Anspruch 5, wobei das lipolytische Mittel ausgewählt ist aus: Phosphodiesterase-Inhibitoren, α-2 Blocker-Verbindungen, β-adrenerge Agonisten und Antagonisten, β-Rezeptoren und/oder G-Proteine stimulierende Verbindungen, Glukose-Transport Blocker, zum Blocken von Neuropeptid Y (NPY)-Rezeptoren auf der Adipozyten-Oberfläche geeignete NPY-Antagonisten, Fettsäuretransport modifizierende Mittel, lipolytische Peptide und lipolytische Proteine.

7. Verwendung nach Anspruch 6, wobei der Phosphodiesterase-Inhibitor ausgewählt ist aus: Xanthin-Basen (Coffein, Theobromin, Aminophyllin, Theophyllin, Xanthin, Analogons und Derivate derselben) und solche enthaltende natürliche Pflanzenextrakte und insbesondere Methylxanthine.

8. Verwendung nach Anspruch 7, wobei der Phosphodiesterase-Inhibitor Coffein und/oder Theobromin ist.

9. Verwendung nach einem der Ansprüche 1 bis 8, wobei das Diterpen in einem Umfang zwischen circa 0,00001% und circa 50% Gewichtsanteil und bevorzugt von circa 0,0001% bis circa 10% Gewichtsanteil und besonders bevorzugt von 0,001% bis circa 1% Gewichtsanteil in Bezug auf das Gewicht der Zusammensetzung vorliegt.

10. Verwendung nach einem der Ansprüche 5 bis 9, wobei das lipolytische Mittel und/oder das Lipogenese inhibierende Mittel in einem Umfang zwischen circa 0,0001% und circa 50% Gewichtsanteil und bevorzugt von circa 0,001% bis circa 10% Gewichtsanteil und besonders bevorzugt von 0,01% bis circa 1% Gewichtsanteil in Bezug auf das Gewicht der Zusammensetzung vorliegt.

11. Verwendung nach Anspruch 7 oder 8, wobei der Xanthin-Basen umfassende natürliche Pflanzenextrakt ein Yerba Mate Extrakt ist.

12. Verwendung nach einem der Ansprüche 1 bis11, weiter umfassend wenigstens einen ergänzenden aktiven Inhaltsstoff ausgewählt aus: auf Mikrozirkulation wirkende Mittel, Festigungsmittel und/oder Anti-Glykationsmittel und Mischungen derselben.

13. Verwendung nach einem der Ansprüche 1 bis12, wobei das Diterpen verwendet wird in der Art einer Lösung, Dispersion, Emulsion, Paste oder Puder, individuell oder in einer Vormischung, oder individuell oder in einer Vormischung getragen wird von Vektoren wie Makrokapseln, Mikrokapseln, Nanokapseln, Makrokugeln, Mikrokugeln, Nanokugeln, Liposome, Oleosome oder Chylomikrone, Makropartikel, Mikropartikel, Nanopartikel, Makroschwämme, Mikroschwämme oder Nanoschwämme oder adsorbiert an organischen Polymer-Pudern, Talk, Bentonit oder anderen anorganischen Trägern.

14. Verwendung nach einem der Ansprüche 1 bis13, wobei das Diterpen individuell verwendet wird oder in einer Vormischung in einem geeigneten Trägermittel in einer beliebigen kosmetischen Form und insbesondere in erweichenden Lotionen, Milchen oder Cremes, Milchen oder Cremes zur Hautpflege, von Make-up reinigenden Milche oder Cremes, Grundierungsmitteln, vor Sonne schützenden Lotionen, Sonnenmilchen oder Cremes, künstlich bräunenden Lotionen, künstlich bräunenden Milchen oder Cremes; Rasurcremes oder Rasurschäumen oder Aftershave Lotionen.

15. Verwendung nach einem der Ansprüche 1 bis14, weiter umfassend wenigstens einen ergänzenden Inhaltsstoff ausgewählt aus den folgenden: organische oder hydroglykolische Lösungsmittel, extrahierte oder synthetische Fette, ionische oder nichtionische Verdickungsmittel, Weichmacher, Trübungsmittel, Stabilisatoren, Silikone, α- oder β-Hydroxysäuren, Konservierungsmittel, Komplexbildner, gelierende und viskosierende Polymere, Tenside und Emulgatoren, Pflanzenextrakte, Gewebeextrakte, Meeresextrakte, Sonnenschutzfilter, Feuchtigkeitsmittel, weichmachende Mittel, Produkte zur Behandlung von Hautkrankheiten, Germizide, Treibmittel, Reinigungsmittel, hautpflegende Mittel, Parfüms, vor Sonne schützende und/oder Sonne blockende Zusammensetzungen für die Haut, Detergenzien, Emolliente, antiseptische Mittel, Abrasive, Absorptionsmittel, ästhetische Mittel wie beispielsweise Duftstoffe, Pigmente, Farbstoffe/Colorationen für die Haut, Make-up-Mittel, ätherische Öle, Hautsensate, Adstringenzien, Anti-Aknemittel, Anti-Backmittel, Schaumverhüter, antimikrobielle Mittel, Bindemittel, biologische Zusätze, Pufferungsmittel, Auflockerungsmittel, Chelatbildner, chemische Zusätze, kosmetische Adstringenzien, kosmetische Biozide, Denaturierungsmittel, pharmakologische Adstringenzien, externe Analgetika, Filmbildner oder Filmmaterial, pH-Regulierer, Reduziermittel, hautbleichende und aufhellende Mittel, Feuchthaltemittel, hautschonende und/oder heilende Mittel, hautbehandelnde Mittel, Peptide und Peptidderivate derselben, Vitamine und Derivate derselben, Netzmittel, künstlich sonnenbräunende Mittel; wasserlösliche Sonnenschutzmittel; Antitranspirante,
Deodorants und erfrischende, tonische, heilungsfördernde, keratolytische und enthaarende Produkte, parfümiertes Wasser, botanische Extrakte, pflanzliche Gewebeextrakte, wasserlösliche Färbemittel, antiseborrhoische Mittel, Oxidationsmittel, Vitamine B1 bis B12, C, D, H, K und deren Derivate, Peptidhormone oder Steroidhormone, Enzyme, Coenzyme, Enzyminhibitoren, Enzymaktivatoren, bakterizide Substanzen, fettlösliche aktive Substanzen, zur Verbesserung des Zustands trockener oder gealterter Haut vorgesehene Substanzen, Tocopherole, Vitamine E, F, oder A und deren Ester, Retinsäuren, Antioxidantien, essenzielle Fettsäuren, Glycyrrhetinsäure, Carotinoide, Ceramide und Pseudo-Ceramide und alle Lipidkomplexe in der Art ähnlich zu denen der natürlichen Ceramide der Haut.

16. Verwendung nach einem der Ansprüche 1 bis15 zur Imprägnierung einer beliebigen Art von Textilien, synthetischen oder natürlichen Fasern, Wolle oder einem beliebigen Material, welches verwendet werden kann zur Herstellung von Kleidung oder Unterbekleidung, aktiven Materialien einer beliebigen Art, Wischtücher, Pflaster, Kompressen, Watten, Wattestäbchen, Verbandsmaterialien, Schwämme zur Make-up Entfernung, Masken und beliebige andere Träger, welche in direkten Kontakt mit der Haut kommen können, um eine kontinuierliche topische Versorgung zu ermöglichen.

## Revendications

1. Utilisation topique cosmétique d'au moins un diterpène choisi parmi le cafestol, le kahweol, leurs analogues et dérivés, pour un traitement amincissant des surcharges pondérales des cuisses et des hanches, un raffermissement cutané, pour prévenir et/ou lutter contre la cellulite ou la peau d'orange et/ou pour affiner les contours du visage.

2. Utilisation selon la revendication 1, **caractérisée en ce que** le diterpène correspond à la formule I : 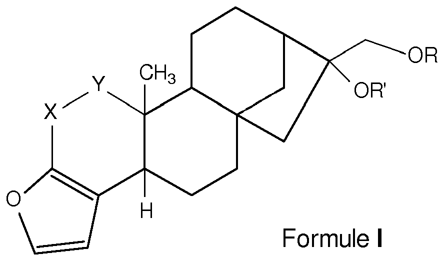 dans laquelle :
- les groupes R et R', qui peuvent être identiques ou différents, représentent chacun un atome d'hydrogène, un groupe alkyle, un groupe aryle, un groupe aralkyle, un groupe acyle, un groupe sulfonyle, ou un groupe sucre ;
- la séquence X-Y correspond ou à CH₂-CH₂ ou à CH=CH, ou à CR¹R²-CR²R⁴, ou encore à CR⁵=CR⁶, dans lesquelles les groupes R¹, R², R³, R⁴, R⁵, R⁶, qui peuvent être identiques ou différents, représentent chacun un atome d'hydrogène, un groupe alkyle, un groupe aryle, un groupe aralkyle, un groupe acyle, un groupe sulfonyle, ou un groupe sucre.

3. Utilisation selon l'une des revendications 1 ou 2, **caractérisée en ce que** ledit diterpène provient d'un extrait de plante.

4. Utilisation selon la revendication 3, **caractérisée en ce que** l'extrait de plante est un extrait de grains de café vert.

5. Utilisation selon l'une quelconque des revendications 1 à 4, comprenant en outre au moins un agent lipolytique et/ou inhibant la lipogénèse, autre que le diterpène.

6. Utilisation selon la revendication 5, **caractérisée en ce que** ledit agent lipolytique est choisi parmi : les inhibiteurs de phosphodiestérase, les composés α-2 bloqueurs, les agonistes et antagonistes β-adrénergiques, les composés stimulant les récepteurs β et/ou les protéines G, les bloqueurs du transport du glucose, les antagonistes du neuropeptide Y (NPY) capables de bloquer les récepteurs NPY à la surface des adipocytes, les agents modifiant le transport des acides gras, les peptides lipolytiques et les protéines lipolytiques.

7. Utilisation selon la revendication 6, **caractérisée en ce que** ledit inhibiteur de phosphodiestérase est choisi parmi : les bases xanthiques (caféine, théobromine, aminophylline, théophylline, xanthine, leurs analogues et dérivés) et les extraits de plantes naturels en contenant, et particulièrement les méthyl xanthines.

8. Utilisation selon la revendication 7, **caractérisée en ce que** ledit inhibiteur de phosphodiestérase est la caféine et/ou la théobromine.

9. Utilisation selon l'une quelconque des revendications 1 à 8, **caractérisée en ce que** ledit diterpène est présent en une quantité comprise entre environ 0,00001% et environ 50% p/p, préférentiellement entre environ 0,0001% et environ 10% p/p, et plus préférentiellement entre environ 0,001% et environ 1% par rapport au poids total de la composition.

10. Utilisation selon l'une quelconque des revendications 5 à 9, **caractérisée en ce que** ledit agent lipolytique et/ou inhibant la lipogénèse est présent en une quantité comprise entre environ 0,0001% et environ 50% p/p, préférentiellement entre environ 0,001% et environ 10% p/p, et plus préférentiellement entre environ 0,01% et environ 1% par rapport au poids total de la composition.

11. Utilisation selon l'une des revendications 7 ou 8, **caractérisée en ce que** l'extrait de plante naturel contenant des bases xanthiques est un extrait de Yerba Maté.

12. Utilisation selon l'une quelconque des revendications 1 à 11, comprenant en outre au moins un actif complémentaire choisi parmi : les actifs agissant sur la micro-circulation, les actifs raffermissants et/ou anti-glycants, et leurs mélanges.

13. Utilisation selon l'une quelconque des revendications 1 à 12, **caractérisée en ce que** ledit diterpène est utilisé sous forme de solution, de dispersion, d'émulsion, de pâte ou de poudre, individuellement ou en pré-mélange, ou est véhiculé individuellement ou en pré-mélange par des vecteurs comme les macrocapsules, microcapsules ou nanocapsules, les macrosphères, microsphères ou nanosphères, les liposomes, oléosomes ou les chylomicrons, les macroparticules, microparticules ou nanoparticules, ou les macroéponges, microéponges, ou nanoéponges, ou est adsorbé sur des polymères organiques poudreux, les talcs, bentonites et autres supports minéraux.

14. Utilisation selon l'une quelconque des revendications 1 à 13, **caractérisée en ce que** ledit diterpène est utilisé individuellement ou en pré-mélange dans un excipient convenable dans toute forme cosmétique, à savoir émollients, lotions, laits ou crèmes, laits ou crèmes pour les soins de la peau, crèmes, lotions ou laits démaquillants, bases de fond de teint, lotions, laits ou crèmes solaires, lotions, laits ou crèmes de bronzage artificiel, crèmes ou mousses de rasage ou lotions après-rasage.

15. Utilisation selon l'une quelconque des revendications 1 à 14, comprenant au moins un ingrédient additionnel choisi parmi : les solvants organiques ou hydroglycoliques, corps gras d'extraction ou de synthèse, épaississants ioniques ou non ioniques, adoucissants, opacifiants, stabilisants, silicones, α- ou β-hydroxyacides, conservateurs, agents séquestrants, polymères gélifiants et viscosants, tensioactifs et émulsifiants, extraits végétaux, extraits tissulaires, extraits marins, filtres solaires, agents hydratants, agents adoucissants, produits pour le traitement d'affections cutanées, germicides, propellants, agents nettoyants, agents de conditionnement de la peau, parfums, écrans solaires et/ou totaux pour la peau, détergents, émollients, agents antiseptiques, abrasifs, absorbants, composants esthétiques comme les parfums, pigments, colorants pour la peau, agents maquillants, huiles essentielles, sensations de peau, astringents, agents anti-acné, agents anti-agglomérants, agents antimousse, agents antimicrobiens, liants, additifs biologiques, agents tampons, agents gonflants, agents chélatants, additifs chimiques, astringents cosmétiques, biocides cosmétiques, dénaturants, astringents pharmaceutiques, analgésiques externes, agents ou matériaux filmogènes, ajusteurs de pH, agents réducteurs, agents blanchissants et éclaircissants de peau, humectants, agents apaisants et/ou soignant la peau, agents de traitement de la peau, peptides et leurs dérivés peptidiques, vitamines et leurs dérivés, agents mouillants, agents de bronzage artificiel; écrans solaires hydrosolubles; antiperspirants,
déodorants, et rafraichissants, produits toniques, cicatrisants, kératolytiques et dépilatoires, eau parfumée, extraits botaniques, extraits de tissus de plantes, colorants hydrosolubles, agents antiseborrhée, agents oxydants, vitamines B1 à B12, C, D, H, K et leurs dérivés, hormones peptidiques ou stéroïdes, enzymes, coenzymes, inhibiteurs d'enzyme, activateurs d'enzyme, substances bactericides, substances actives liposolubles, substances destinées à améliorer l'état de la peau sèche ou âgée, tocophérols, vitamines E, F ou A et leurs esters, acide rétinoïque, antioxydants, acides gras essentiels, acide glycyrrhétinique, caroténoïdes, céramides et pseudocéramides, et tous les complexes lipidiques de formes semblables à celles des céramides naturels de la peau.

16. Utilisation selon l'une quelconque des revendications 1 à 15, pour l'imprégnation de toute sorte de textile, fibre synthétique ou naturelle, laine ou tout matériau susceptible d'être utilisé pour la fabrication de vêtement ou de sous-vêtement, de matériaux actifs de toute sorte, des lingettes, patches, compresses, cotons, cotons-tiges, pansements, éponges de démaquillage, masques et tout autre support susceptible d'entrer en contact direct avec la peau pour permettre une délivrance topique continue.
